# EUROPÄISCHE PATENTSCHRIFT

(11) **EP 0 567 429 B1**
(45) Veröffentlichungstag und Bekanntmachung des Hinweises auf die Patenterteilung: **10.01.1996**
(21) Anmeldenummer: 93810268.8
(22) Anmeldetag: 15.04.1993
(51) Int. Cl.: C07C 323/30

(54) **Substituierte Tetracyanochinodimethane, Verfahren zu deren Herstellung und deren Verwendung als pi-akzeptoren und elektrische Halbleiter**
Substituted tetracyanoquinodimethanes, method for their preparation and their use as pi-acceptors and electrical semi-conductors
Tétracyanoquinodiméthanes substitués, méthode pour leur préparations et leur utilisation comme pi-accepteurs et semi-conducteur électronique

(30) Priorität: 22.04.1992 CH 1305/92
(43) Veröffentlichungstag der Anmeldung: 27.10.1993
(73) Patentinhaber: CIBA-GEIGY AG, CH-4002 Basel (CH)
(72) Erfinder: Zambounis, John, Dr., CH-3280 Murten (CH)

(56) Entgegenhaltungen:
- JOURNAL OF ORGANIC CHEMISTRY, Bd. 40, Nr. 21, 17. Oktober 1975, Washington, DC, US, Seiten 3101 - 3109 R.C. WHELAND, ET AL.: 'Synthesis of substituted 7,7,8,8-tetracyano-quinodimethanes'
- JOURNAL OF THE AMERICAN CHEMICAL SOCIETY, Bd. 98, Nr. 13, 23. Juni 1976, Washington, DC, US, Seiten 3916 - 3625 R.C. WHELAND, ET AL.: 'Synthesis of electrically conductive organic solids'

## Beschreibung

Die vorliegende Erfindung betrifft 1,4-Tetracyanochinodimethane, die in den 2,5-Stellungen mit organischen Thioresten substituiert sind, ein Verfahren zu deren Herstellung und deren Verwendung, und als Zwischenprodukt des Verfahrens in den 2,5-Stellungen mit organischen Thioresten substituiertes 1,4-Di(chlormethyl)-phenyl.

R.C. Wheland und E.L. Martin beschreiben im J. Org. Chem. Vol. 40, Nr. 21, Seiten 3101-9 (1975) substituierte Tetracyanochinodimethane (nachstehend als TCNQ abgekürzt), z.B. TCNQ(O-Ethyl)(S-Methyl), sowie deren Anionsalze. Es ist bekannt, z.B. vom R.C. Whelend et al. in J. Am. Chem. Soc. 98, S.3916-25 (1976) beschrieben, dass unsubstituierte und substituierte TCNQ Elektronenakzeptoren für elektrisch leitende Charge-Transfer (CT) Komplexe verwendet werden können. TCNQ's, die für sich elektrische Leiter oder Halbleiter darstellen, sind noch nicht bekannt.

Es wurde nun überraschend gefunden, dass 1,4-Tetracyanochinodimethane, die in den 2,5-Stellungen mit organischen Thioresten substituiert sind, in Form von Schichten auf einem Trägermaterial für sich elektrische Halbleiter darstellen.

Ein Gegenstand der Erfindung sind Verbindungen der Formel I 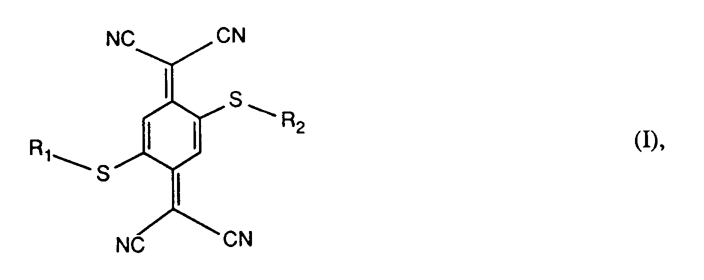
worin
R₁ und R₂ unabhängig voneinander C₁-C₆-Alkyl, unsubstituiertes oder mit C₁-C₄-Alkyl substituiertes C₃-C₈-Cycloalkyl, oder unsubstituiertes oder mit F, Cl, Br, C₁-C₆-Alkyl, C₁-C₆-Alkoxy oder Di(C₁-C₆-Alkyl)amino substituiertes Phenyl oder Benzyl bedeuten. Bei R₁ und R₂ handelt es sich bevorzugt um gleiche Reste.

R₁ und R₂ können als Alkyl linear oder verzweigt sein und bevorzugt 1 bis 4 C-Atome enthalten. Beispiele sind Methyl, Ethyl, n- und i-Propyl, n-, i- und t-Butyl, Pentyl und Hexyl. Bevorzugte Alkylreste sind Methyl und Ethyl.

Beispiele für Cycloalkyle sind Cyclopropyl, Cyclopentyl, Cyclohexyl, Cycloheptyl und Cyclooctyl. Bevorzugte Cycloalkylreste sind Cyclopentyl und Cyclohexyl. Beispiele für substituierte Cycloalkylreste sind Methylcyclopentyl und Methylcyclohexyl.

Beispiele für Alkylsubstituenten, die bevorzugt 1-4 C-Atome enthalten, sind Methyl, Ethyl, n- und i-Propyl, und n-, i- und t-Butyl.

Beispiele für Alkoxysubstituenten, die bevorzugt 1-4 C-Atome enthalten, sind Methoxy, Ethoxy, n-Propoxy und t-Butoxy.

Beispiele für Dialkylamino-Substituenten, worin die Alkylgruppen bevorzugt 1-4 C-Atome enthalten, sind Dimethylamino, Diethylamino und Di-n-propylamino.

Einige Beispiele für substituiertes Phenyl und Benzyl sind 2,6-Dimethylphen-4-yl, 2,6-Diethylphen-4-yl, Toluyl, p-Methoxyphenyl und 2,6-Dimethoxyphen-4-yl.

R₁ und R₂ stellen bevorzugt unsubstituiertes C₁-C₄-Alkyl, Phenyl oder Benzyl dar.

Bevorzugte Beispiele für R₁ und R₂ sind Methyl, Ethyl, n-Propyl, Cyclopentyl, Cyclohexyl, Phenyl und Benzyl.

Besonders bevorzugt sind R₁ und R₂ gleich und stehen für Methyl. Es handelt sich dann um die Verbindung 2,5-Bis(thiomethyl)-1,4-tetracyanoquinodimethan, nachstehend als 2,5-DMeS-TCNQ abgekürzt.

Ein anderer Gegenstand der Erfindung ist ein Verfahren zur Herstellung von Verbindungen der Formel I, das dadurch gekennzeichnet ist, dass man
i) eine Verbindung der Formel II 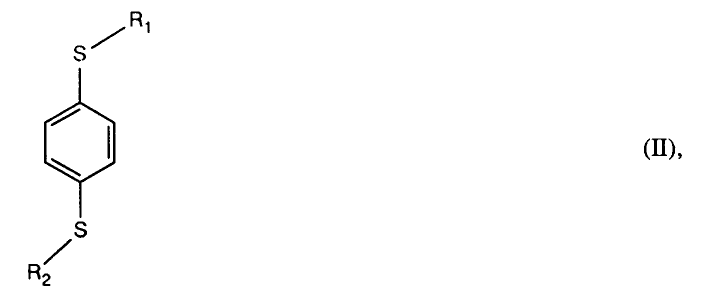 worin R₁ und R₂ die zuvor angegebenen Bedeutungen haben, in Gegenwart von konzentrierter HCl und CH₃COOH mit Paraformaldehyd zu einer Verbindung der Formel III 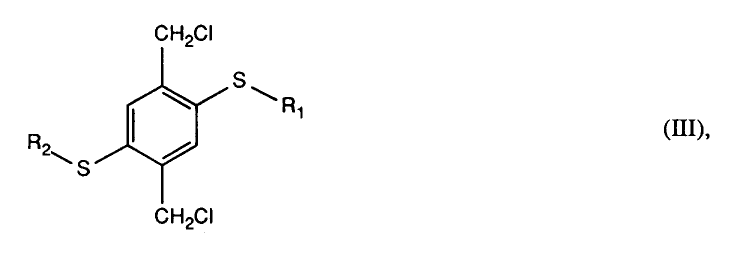 umsetzt,
ii) diese Verbindung der Formel III durch Umsetzung mit NaCN in eine Verbindung der Formel IV 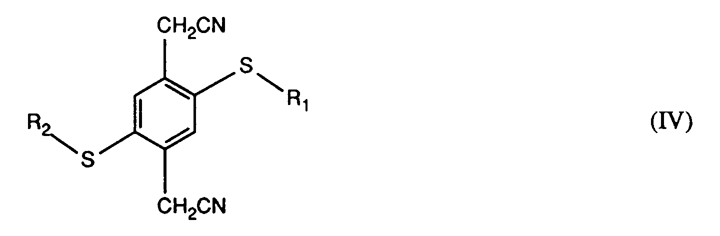 umwandelt,
iii) diese Verbindung der Formel IV dann durch Umsetzung mit CH₃ONa, (CH₃O)₂CO und ClCN in eine Verbindung der Formel V 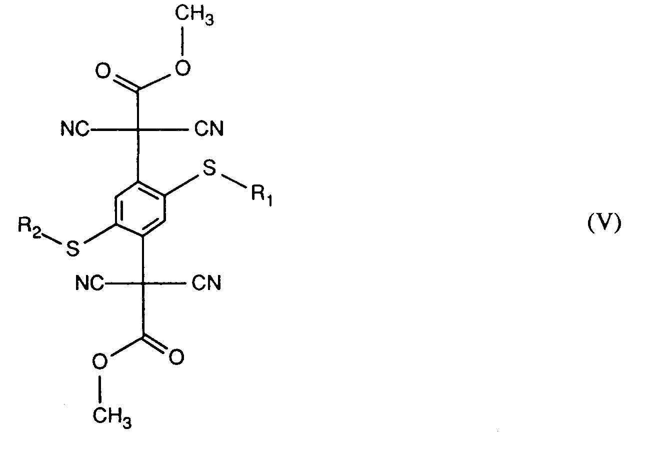 überführt, und
iv) diese Verbindung der Formel V in einer wässrigen Base unter inerter Atmosphäre löst, und durch Einwirkung von Br₂ und konzentrierter HCl in die Verbindung der Formel I umsetzt.

Das Verfahren ist an sich bekannt und in J. Org. Chem. Vol. 40, Nr. 21, S. 3101-9 (1975) von R.C. Wheland und E.L. Martin beschrieben.

Ferner ist das Verfahren zur Herstellung der Ausgangsprodukte der Formel II an sich bekannt, und in J. Organometallic Chem., 296, S. 357-66 (1985) von L. Engman et al. beschrieben.

Die Reaktionsstufen i) und iv) können bei einer Reaktionstemperatur zwischen 40 und 110°C, vorzugsweise 50 bis 90°C, durchgeführt werden.

Geeignete Lösungsmittel für Reaktionsstufe ii) können polar und aprotisch sein, und schliessen z.B. folgende ein: Sulfone; Sulfoxide; N,N'-tetrasubstituierte Harnstoffe; N-alkylierte Lactame oder N-dialkylierte Säureamide; Ether; aliphatische, cycloaliphatische oder aromatische Kohlenwasserstoffe, die mit F, Cl oder C₁-C₄-Alkyl substituiert werden können; Carbonsäureester und Lactone; Nitrile.

### Beispiele sind:

Sulfone: Dimethylsulfon, Diethylsulfon.
Sulfoxide: Dimethylsulfoxid (DMSO), Diethylsulfoxid.
N,N-Tetrasubstituierter Harnstoff: N-Methylethyl-N'-methylethylharnstoff, Tetramethylharnstoff.
N-Alkylierte Lactame: N-Methylpyrrolidon, N-Ethylpyrrolidon.
N-Dialkylierte Säureamide: N-Dimethylformamid, N-Dimethylacetamid.
Ether: Diethylenglykoldimethylether, Diethylenglykoldiethylether.
Aliphatische Kohlenwasserstoffe: Hexan, Heptan, Nonan.
Cycloaliphatische Kohlenwasserstoffe: Decahydronaphtalin, Methylcyclohexan.
Aromatische Kohlenwasserstoffe: Xylol, Benzol, Dichlorbenzol.
Carbonsäureester: Essigsäuremethylester, Essigsäureethylester.
Nitrile: Benzonitril, Phenylacetonitril, Acetonitril.

Bevorzugte Lösungsmittel sind DMSO und Dimethylformamid.

Die Zugabe des NaCN kann bei 20 bis 30°C erfolgen und nach der vollständigen Zugabe kann die Temperatur zwischen 40 und 90°C gehalten werden.

Bei der Stufe iii) können CH₃ONa, (CH₃O)₂CO mit einer Verbindung der Formel IV zusammengegeben und zum Beispiel bei 50 bis 80°C umgesetzt werden. Ein mit CH₃OH ein Azeotrop bildendes Lösungsmittel, z.B. Benzol, kann zugegeben werden, um das gebildete Methanolazeotrop abzudestillieren. Danach kann ClCN bei 0-10°C, vorzugsweise 3 bis 7°C eingeleitet werden, bis die Umsetzung vollständig ist.

Die letzte Stufe, iv), wird zweckmässig unter einer inerten Atmosphäre, z.B. Argon, durchgeführt, wobei die Verbindung der Formel V zunächst in KOH oder NaOH gelöst wird. Die Reaktion mit Br₂ und konzentrierter Säure, z.B. HCl, wird vorteilhaft bei Raumtemperatur durchgeführt. Bei dieser Verfahrensstufe können zusätzlich mit Wasser mischbare Lösungsmittel verwendet werden, zum Beispiel Methanol, Ethanol, Diethylether, Tetrahydrofuran und Dioxan.

Das Produkt jeder Stufe kann in an sich bekannter Weise isoliert werden, z.B. durch Dekantieren, Filtration oder Destillation. Anschliessend können die Produkte mittels üblicher Methoden wie zum Beispiel Kristallisation oder chromatographischer Methoden gereinigt werden.

Ein anderer Gegenstand der Erfindung betrifft Verbindungen der Formel III, 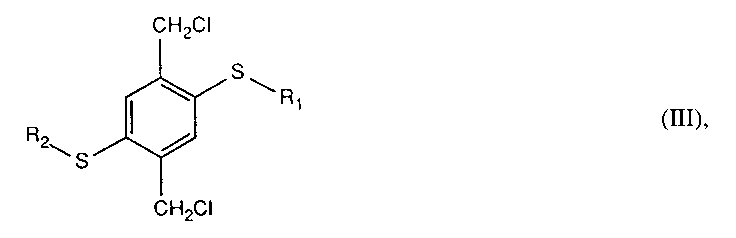
worin
R₁ und R₂ den zuvor angegebenen Definitionen entsprechen.

Besonders bevorzugt sind R₁ und R₂ gleich und stehen für Methyl. Es handelt sich dann um die Verbindung 1,4-Bis(chloromethyl)-2,5-bis(thiomethyl)-benzol.

Die Verbindungen der Formel I sind pi-Akzeptoren und werden durch Luft nicht oxidiert. Als aufgedampfte Schichten zeigen sie elektrische Leitfähigkeiten, die denen von Halbleitern entsprechen. Die erfindungsgemässen Verbindungen der Formel I sind überraschend für sich organische Halbleiter, die aus nur einer Komponente aufgebaut sind, und diese Eigenschaft überraschend im Kontakt mit Luft nicht verändern.

Ein weiterer Gegenstand der Erfindung ist ein Trägermaterial, das auf mindestens einer Oberfläche eine Schicht mindestens einer Verbindung der Formel I enthält.

Geeignete Träger sind zum Beispiel Metalle, Metallegierungen, Gläser, Mineralien, Keramiken und duroplastische oder thermoplastische Kunststoffe. Bevorzugt sind Gläser und duroplastische oder thermoplastische Kunststoffe. Der Träger kann eine Dicke von 0,01 mm bis 1 cm, bevorzugt 0,1 mm bis 0,5 cm aufweisen. Bevorzugte Träger sind Gläser und homo- oder kopolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze.

Träger können nach den für Duroplasten und Thermoplasten üblichen Misch- und Form-gebungsverfahren wie zum Beispiel Giess-, Press-, Spritzguss- und Extrusionsverfahren hergestellt werden.

Auf dem Träger können eine oder mehrere Schichten aus einem inerten isolierenden Material, z.B. Kunststoffen, als Zwischenschicht(en) aufgebracht sein, besonders wenn der Träger ein elektrisch leitender Werkstoff ist. Die Zwischenschichten können auch als Haftvermittler dienen.

Die Schichtdicke des inerten isolierenden Materials beträgt zum Beispiel 40 bis 3000 Å, bevorzugt 50 bis 2000 Å.

Auf dem Träger bzw. der Zwischenschicht können eine oder mehrere, zum Beispiel 1 bis 10, bevorzugt 1 bis 5 und besonders bevorzugt 1 bis 3 Schichten aus Verbindungen der Formel I aufgebracht sein.

Die Schichtdicke der Schicht aus Verbindungen der Formel I beträgt zum Beispiel 100 bis 3000 Å, bevorzugt 100 bis 2000 Å und besonders bevorzugt 200 bis 1500 Å.

Auf der Schicht aus Verbindungen der Formel I kann eine Schutzschicht aufgebracht sein, z.B. aus Kunststoffen.

Geeignete Beschichtungsverfahren sind zum Beispiel Tauchen, Giessen, Streichen, Rakeln, Schleudergiessen und Aufdampfverfahren, die im Hochvakuum durchgeführt werden. Bei der Verwendung von Lösungsmitteln ist darauf zu achten, dass die verwendeten Träger gegen diese Lösungsmittel unempfindlich sind. Bevorzugt werden die Schichten mittels Aufdampfverfahren besonders im Vakuum hergestellt. Geeignete Beschichtungsverfahren sind zum Beispiel in der EP-A-0 401 791 beschrieben.

Das Aufbringen der Schicht(en) erfolgt bevorzugt durch Aufdampfen im Vakuum. Das aufzubringende Material wird zunächst in ein geeignetes Gefäss gefüllt, das gegebenenfalls mit einer Widerstandsheizung versehen ist, und dann in eine Vakuumkammer gesetzt. Der zu bedampfende Träger wird oberhalb des Gefässes mit dem aufzudampfenden Material in eine Halterung eingesetzt. Diese ist so konstruiert, dass der Träger gegebenenfalls rotiert (z.B. mit 10 Upm) und geheizt werden kann. Die Vakuumkammer wird auf etwa 1,3 · 10⁻⁵ bis 1,3 · 10⁻⁶ mbar (10⁻⁵ bis 10⁻⁶ Torr) evakuiert, und die Heizung wird so eingestellt, dass die Temperatur des aufzudampfenden Materials bis zu dessen Verdampfungstemperatur ansteigt. Die Verdampfung wird so lange fortgesetzt, bis die aufgedampfte Schicht die gewünschte Dicke hat.

Die erfindungsgemässen Verbindungen der Formel I bilden festhaftende Schichten auf den Trägermaterialien. Auf Grund seiner Halbleitereigenschaften eignet sich das beschichtete Trägermaterial hervorragend zur Abschirmung von elektrischen und/oder magnetischen Feldern. Ein weiterer Gegenstand der Erfindung ist die Verwendung des erfindungsgemässen beschichteten Materials als antistatisches Verpackungsmaterial.

Ein anderer Gegenstand der Erfindung ist die Verwendung der Verbindungen der Formel I zur antistatischen Ausrüstung von Gegenständen.

Die erfindungsgemässen Verbindungen der Formel I können Charge-Transfer (CT) Komplexe mit den folgenden Kationen bilden: z.B. Ag, Cu, Ni, Alkalimetallen, z.B. Li, Na, K, Erdalkalimetallen, z.B. Mg, Ca, Sr, sowie mit anderen Elektronenakzeptoren wie z.B. Tetrathiofulvalenen, Tetrathioteoacenen, N-Alkylpyridinen, Chinolinen und Ferrocenen. Ähnliche CT-Komplexe sind z.B. vom I.A. Howard im Semiconductors and Semimetals, Vol.27, S.29-85 (1988) beschrieben.

Die nachfolgenden Beispiele erläutern die Erfindung näher.

### A. Herstellungsbeispiel

### Beispiel A1:

### a) Herstellung von 1,4-Bis(chloromethyl)-2,5-bis(thiomethyl)-benzol

Eine Suspension von 21,5 g (126 mMol) 1,4-Bis(thiomethyl)-benzol in einer Lösung von 60 ml HCl (37%) und 60 ml CH₃COOH wird im 100 °C heissem Ölbad erwärmt. Bei 50 °C Innentemperatur werden 19 g (633 mMol) Paraformaldehyd zugegeben und bei 65 °C Innentemperatur HCl gasförmig durchgeleitet. Das Reaktionsgemisch bildet bei dieser Temperatur eine Emulsion. Die Innentemperatur erhöht sich auf 85 °C und nach 30 Minuten bilden sich feste Anteile. Während der nächsten Stunde wird bei intensivem Rühren HCl durch das Reaktionsgemisch geleitet. Anschliessend wird das heisse Reaktionsgemisch abdekantiert und mit Wasser gewaschen. Zum Rückstand wird 100 ml Diethylether gegeben, abfiltriert und die erhaltene Subtanz mit Diethylether und danach mit Wasser gewaschen und im Vakuum getrocknet: Ausbeute 5,9 g (17,5%) weisse Kristalle, Schmelzpunkt (Smp.): 189 bis 189,5 °C. MS (m/e): 266 (M⁺, 100%), NMR (CDCl₃): 2,52 (3H, s), 4,73 (2H, s), 7,34 (2H, s).

### b) Herstellung von 1,4-Bis(cyanomethyl)-2,5-bis(thiomethyl)-benzol

2,6 g (53 mMol) NaCN werden in DMSO suspendiert und unter Rühren auf 50°C erwärmt. Nach ca. 20 Minuten ist alles gelöst. Das Heizbad wird abgesenkt und 5,9 g (22 mMol) 1,4-Bis(chloromethyl)-2,5-bis(thiomethyl)-benzol (Beispiel A1a) in Portionen eingetragen; die Reaktion ist exotherm, die Temperatur wird zwischen 47 und 50 °C gehalten. Dauer des Eintrages: 25 Minuten. Danach wird 1 h bei 50 bis 55°C und 10 Minuten bei 85°C nachgerührt. Nach dem Heizen wird auf 40 °C abgekühlt und die Lösung in 350 ml kaltes Wasser gegossen. Der erhaltene Niederschlag wird abgenutscht und mit Wasser neutral gewaschen. Das erhaltene Produkt mit 50 ml Ethanol nachgewaschen und getrocknet. Ausbeute: 5,5 g (100%) weisse Kristalle, Smp.: 198 bis 205°C. MS (m/e): (M⁺, 100%), NMR (CDCl₃): 2,53 (3H, s), 3,83 (2H, s), 7,38 (2H, s).

### c) Herstellung von Dimethyl α,α,α',α'-tetracyano-2,5-bis(thiomethyl)-1,4-phenylendiacetat

Eine Suspension von 5,5 g (22 mMol) 1,4-Bis(cyanmethyl)-2,5-bis(thiomethyl)-benzol (Beispiel A1b) in 30 ml Dimethylcarbonat wird unter gutem Rühren mit 2,96 g (55 mMol) frisch hergestelltem CH₃ONa versetzt. Die Suspension wird im 100 °C warmen Ölbad erwärmt. Dabei wird sie braun, das Edukt geht in Lösung und fällt kurz darauf als feiner Niederschlag wieder aus. Bei 65 °C werden 21 ml Benzol zugegeben und das sich bildende Methanol azeotrop abdestilliert. Dieser Vorgang wird innerhalb 2 h viermal wiederholt. Am Ende ist der Siedepunkt bei 78°C. Es wird nochmals mit 21 ml Benzol verdünnt und die Suspension mit Eis auf 5 °C abgekühlt. Danach werden 7,5 ml zuvor kondensiertes ClCN eingeleitet, wobei eine leicht exotherme Reaktion stattfindet. Nach dem Einleiten wird das Eisbad entfernt und die hellbraune Suspension auf Raumtemperatur (RT) erwärmt. Die Suspension wird dann 2 h bei 70 °C gerührt. Anschliessend wird im Vakuum zur Trockne eingeengt. Der Rückstand wird mit H₂O verührt, abdekantiert, mehrmals mit frischem Wasser behandelt, mit Ethanol zur Kristallisation gebracht, abgenutscht, mit frischem Ethanol nachgewaschen und getrocknet. Ausbeute: 4,4 g (47,9%) weisses Pulver, Smp.: 172 bis 177°C. MS (m/e): 414 (M⁺, 61%), NMR (CDCl₃): 2,60 (3H, s), 4,06 (3H, s), 7,94 (2H, s).

### d) Herstellung von 2,5-Bis(thiomethyl)tetracyanoquinodimethan (2,5-DMeS-TCNQ)

Eine Suspension von 4,4 g (10,6 mMol) Dimethyl-α,α,α',α'-tetracyano-2,5-bis(thiomethyl)-1,4-phenylendiacetat (Beispiel A1c) in 25 ml 10% KOH wird im 60 °C warmen Ölbad unter Argon während 30 min erwärmt und danach auf RT innerhalb 1 h abgekühlt. Die Suspension wird abgenutscht und das rotbraune Filtrat zu einer Lösung von 15 ml konzentrierter HCl in 10 ml Wasser unter Kühlen zugegeben, wobei ein hellbeiger Niederschlag entsteht. Diese Suspension wird mit einer Lösung von 2,1 g (13 mMol) Br₂ in 110 ml Wasser versetzt. Die vorhandene dunkelbraun-schwarze Suspension wird nach 15 min abgenutscht und mit Wasser gewaschen. Nach wiederholtem Waschen mit 80 ml Ethanol und 80 ml Aceton wird das Rohprodukt in 2,5 l CH₂Cl₂ warm gelöst und danach auf ein Volumen von 600 ml eingedampft. Die dunkelgrüne Lösung wird über 700 gr Kieselgel mit CH₂Cl₂ filtriert. Das Rohprodukt wird im Quarzrohr bei einer Ofentemperatur von 210 bis 215 °C unter 10⁻² mbar Vakuum sublimiert. Ausbeute: 1,24 gr (39,6%) gold-braune Kristalle, Smp.:305,4°C. MS (m/e): 296 (M⁺, 79%), NMR (CDCl₃): 2,64 (3H, s), 7,02 (2H, s).

### B. Anwendungsbeispiele

### Beispiel B1:

### Leitfähigkeitsmessungen

Auf ein Glassubstrat wird eine 100 Å dünne SiO₂-Schicht aufgedampft, gefolgt von einer 380 ± 40 Å dünnen 2,5-DMeS-TCNQ Schicht. Mit Hilfe einer Aufdampfmaske werden vier 0,6 mm breite Goldstreifen mit im Abstand von 1 mm auf die leitende Schicht appliziert. Mit diesem Probenaufbau wird eine spezifische Leitfähigkeit von σ=1,9 10⁻⁵ S/cm bestimmt. Das von R. Wheland et al. im J. Org. Chem. 10, Nr.21, S.3101 (1975) beschriebene TCNQ(OEt)(SMe) zeigt unter identischen Bedingungen eine Leitfähigkeit von σ=1,8 10⁻⁷ S/cm.

### Messung der Oberflächenentladung:

Die Oberflächenentladung von etwa 200V wird mit Hilfe eines goldbeschichteten Wolframdrahts von 50 Micrometer Durchmesser gebildet, der mit einer Spannung von ungefähr 3,4 kV bei einer Luftfeuchtigkeit von 1,3% aufgeladen wird. Diese Spannung ist so geregelt, dass der Strom bei konstant 200 nA pro cm Drahtlänge gehalten wird. Die Probe wird mit Silberpaste auf einen Glasträger geklebt und mit einer Kontaktspitze am Rand des Trägers elektrisch verbunden. Während eines Messverlaufs verschiebt sich der Träger 8 mm unter dem Coronadraht mit einer Geschwindigkeit von etwa 50 cm/s, und hält an dem Punkt an, wo die Kontaktspitze in einen geerdeten leitfähigen Schaum eintaucht. Die Probe liegt dann unter einem Feldmeter (Isoprobe Electrostatic Voltmeter 244, Monroe Electronics Inc.). Die Spannung der aufgedampften Schicht wird 5 Sekunden nach der Coronaaufladung gemessen und ergibt 2 ± 1 Volt.

### Messungen der Transmittance

Die Transmittance der aufgedampften Schicht gemessen im Bereich 300-900 nm ist grösser als 43%.

### Beispiel B2:

Auf eine Polyesterfolie wird eine 570±40 Å dünne 2,5-DMeS-TCNQ Schicht aufgedampft. Die spezifische Leitfähigkeit, gemessen wie in Beispiel B1 beschrieben, beträgt 10⁻⁶ S/cm. Die Spannung dieser aufgedampften Schicht wird 5 Sekunden nach der Coronaaufladung gemessen und ergibt 2 ± 1 Volt.

### Beispiel B3: 2,5-DMeS-TCNQ als Elektronenakzeptor für die Herstellung von organischen Metallen

In einer mit zwei 0.5 mm ⌀ Pt-Elektroden ausgerüsteten, 5 ml fassenden Elektrolysezelle (Abstand der Elektroden voneinander: 2 cm) werden 8 mg 2,5-DMeS-TCNQ und 40 mg AgBF₄ in 3 ml CH₃CN bei einer Stromstärke von 2 µA über 30 Tage elektrolysiert. Die 0.5 mm langen, dunkel-blauen Nadeln, die an der Kathode abgeschieden werden, besitzen eine Leitfähigkeit von ca. σ=2 10⁻³ S/cm. Die Formel von diesem CT-Komplex kann allgemein als Agₓ(2,5-DMeS-TCNQ) beschrieben werden.

## Patentansprüche

1. Verbindungen der Formel I 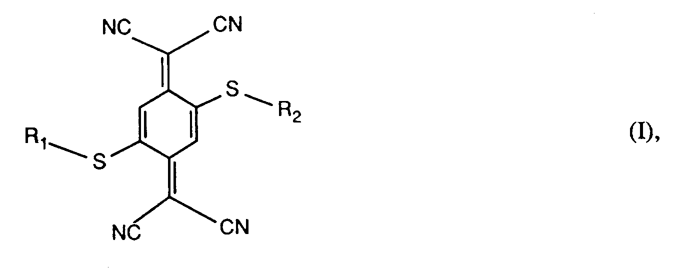 worin
R₁ und R₂ unabhängig voneinander C₁-C₆-Alkyl, unsubstituiertes oder mit C₁-C₄-Alkyl substituiertes C₃-C₈-Cycloalkyl, unsubstituiertes Phenyl oder Benzyl, oder mit F, Cl, Br, C₁-C₆-Alkyl, C₁-C₆-Alkoxy oder Di(C₁-C₆-Alkyl)amino substituiertes Phenyl oder Benzyl bedeuten.

2. Verbindungen der Formel I gemäss Anspruch 1, worin R₁ und R₂ gleich sind.

3. Verbindungen der Formel I gemäss Anspruch 1, worin R₁ und R₂ unabhängig voneinander C₁-C₄-Alkyl sind.

4. Verbindungen der Formel I gemäss Anspruch 3, worin R₁ und R₂ Methyl, Ethyl, n-Propyl, Cyclopentyl, Cyclohexyl, Phenyl oder Benzyl bedeuten.

5. Eine Verbindung der Formel I gemäss Anspruch 1, dadurch gekennzeichnet, dass es sich um 2,5-Bis(thiomethyl)-tetracyanochinodimethan handelt.

6. Verfahren zur Herstellung von Verbindungen der Formel I, dadurch gekennzeichnet, dass man
i) eine Verbindung der Formel II 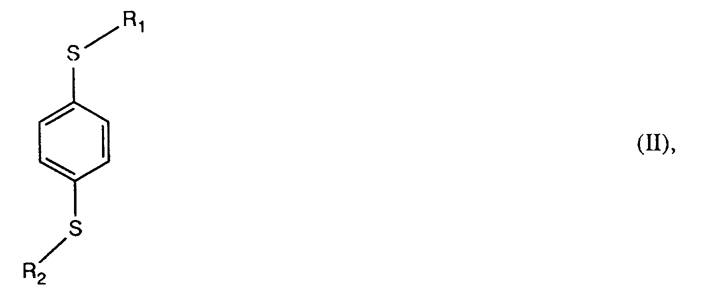 worin R₁ und R₂ die zuvor angegebenen Bedeutungen haben, in Gegenwart von konzentrierter HCl und CH₃COOH mit Paraformaldehyd zu einer Verbindung der Formel III 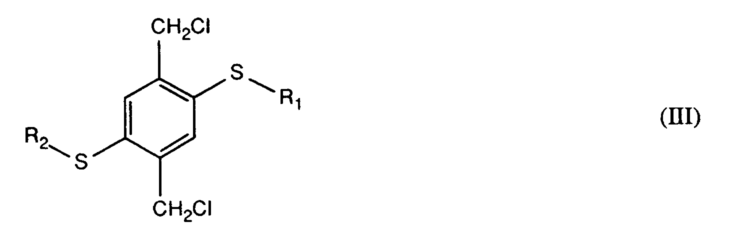 umsetzt,
ii) diese Verbindung der Formel III durch Umsetzung mit NaCN in eine Verbindung der Formel IV 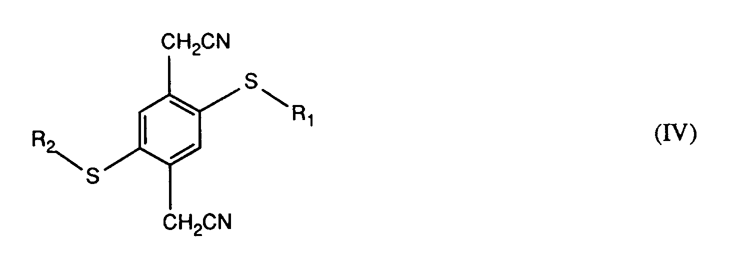 umwandelt,
iii) diese Verbindung der Formel IV dann durch Umsetzung mit CH₃ONa, (CH₃O)₂CO und ClCN in eine Verbindung der Formel V 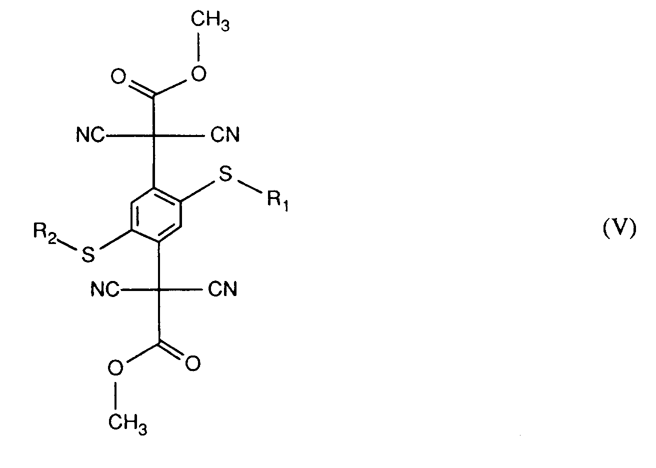 überführt, und
iv) diese Verbindung der Formel V in einer wässrigen Base unter inerter Atmosphäre löst, und durch Einwirkung von Br₂ und konzentrierter HCl in die Verbindung der Formel I umsetzt.

7. Verbindungen der Formel III 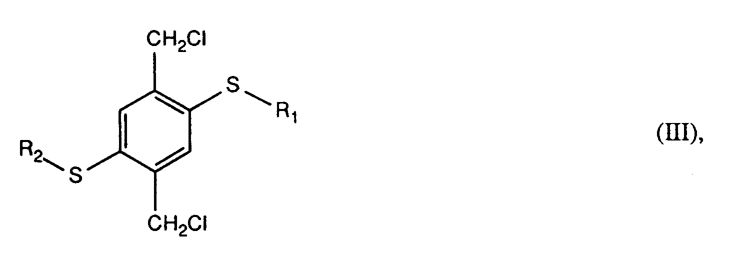 worin
R₁ und R₂ die im Anspruch 1 angegebenen Bedeutungen haben.

8. Eine Verbindung der Formel III gemäss Anspruch 7, dadurch gekennzeichnet, dass es sich um 1,4-Bis(chloromethyl)-2,5-bis(thiomethyl)-benzol handelt.

9. Trägermaterial, das auf mindestens einer Oberfläche eine Schicht mindestens einer Verbindung der Formel I gemäss Anspruch 1 enthält.

10. Material gemäss Anspruch 9, dadurch gekennzeichnet, dass es sich bei dem Träger um Metalle, Metallegierungen, Gläser, Mineralien, Keramiken und duroplastische oder thermoplastische Kunststoffe handelt.

11. Material gemäss Anspruch 9, dadurch gekennzeichnet, dass es sich bei dem Träger um Gläser und homo- oder kopolymere Kunststoffe handelt.

12. Material gemäss Anspruch 9, dadurch gekennzeichnet, dass der Träger eine Dicke von 0,01 mm bis 1 cm aufweist.

13. Material gemäss Anspruch 9, dadurch gekennzeichnet, dass es sich bei dem Kunststoff um thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze handelt.

14. Material gemäss Anspruch 9, dadurch gekennzeichnet, dass sich mindestens eine Zwischenschicht aus einem inerten isolierenden Material zwischen dem Träger und der Schicht aus Verbindungen der Formel I befindet.

15. Material gemäss Anspruch 14, dadurch gekennzeichnet, dass es sich bei der Zwischenschicht um Kunststoffe handelt.

16. Material gemäss Anspruch 14, dadurch gekennzeichnet, dass die Schichtdicke der Zwischenschicht(en) 40 bis 3000 Å beträgt.

17. Material gemäss Anspruch 9, dadurch gekennzeichnet, dass auf dem Träger bzw. der Zwischenschicht 1 bis 10 Schichten aus Verbindungen der Formel I aufgebracht sind.

18. Material gemäss Anspruch 17, dadurch gekennzeichnet, dass die Schichtdicke der Schicht(en) aus Verbindungen der Formel I 100 bis 3000 Å beträgt.

19. Material gemäss Anspruch 9, dadurch gekennzeichnet, dass sich auf der/den Schicht(en) aus Verbindungen der Formel I eine Schutzschicht befindet.

20. Verwendung der Verbindungen der Formel I gemäss Anspruch 1 zur antistatischen Ausrüstung von Gegenständen.

21. Verwendung des Materials gemäss Anspruch 9 als antistatisches Verpackungsmaterial.

## Claims

1. A compound of the formula I 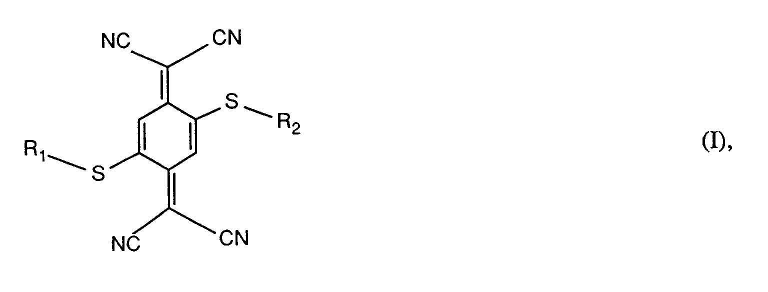 in which
R₁ and R₂ independently of one another are C₁-C₆alkyl, C₃-C₈cycloalkyl which is unsubstituted or substituted by C₁-C₄alkyl, unsubstituted phenyl or benzyl, or phenyl or benzyl which is substituted by F, Cl, Br, C₁-C₆alkyl, C₁-C₆alkoxy or di(C₁-C₆alkyl)amino.

2. A compound of the formula I according to claim 1, in which R₁ and R₂ are identical.

3. A compound of the formula I according to claim 1, in which R₁ and R₂ independently of one another are C₁-C₄alkyl.

4. A compound of the formula I according to claim 3, in which R₁ and R₂ are methyl, ethyl, n-propyl, cyclopentyl, cyclohexyl, phenyl or benzyl.

5. A compound of the formula I according to claim 1, which is 2,5-bis(thiomethyl)-tetracyanoquinodimethane.

6. A process for the preparation of the compound of the formula I, which comprises
i) reacting a compound of the formula II 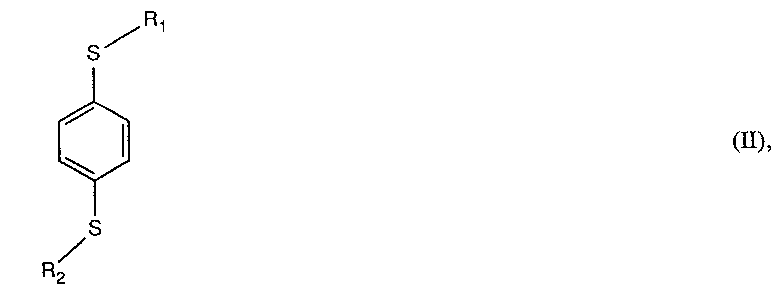 in which R₁ and R₂ are as defined above, with paraformaldehyde in the presence of concentrated HCl and CH₃COOH to give a compound of the formula III 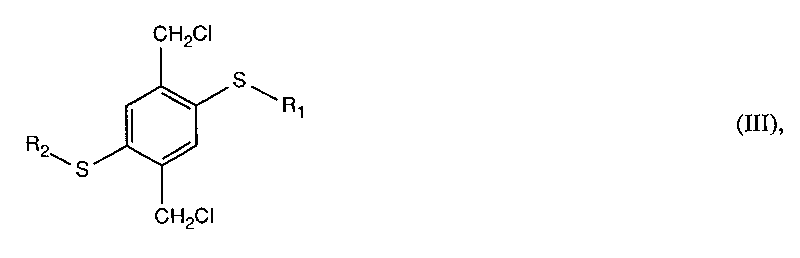
ii) converting this compound of the formula III into a compound of the formula IV 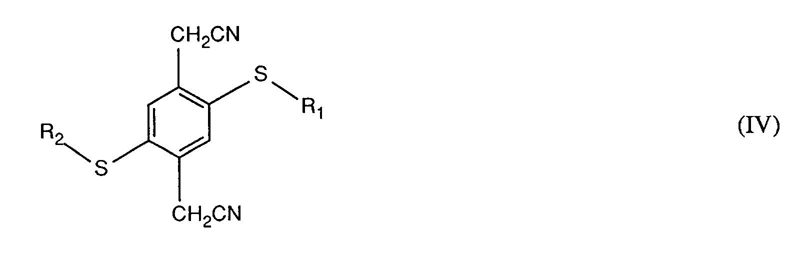 by reaction with NaCN,
iii) subsequently converting this compound of the formula IV into a compound of the formula V 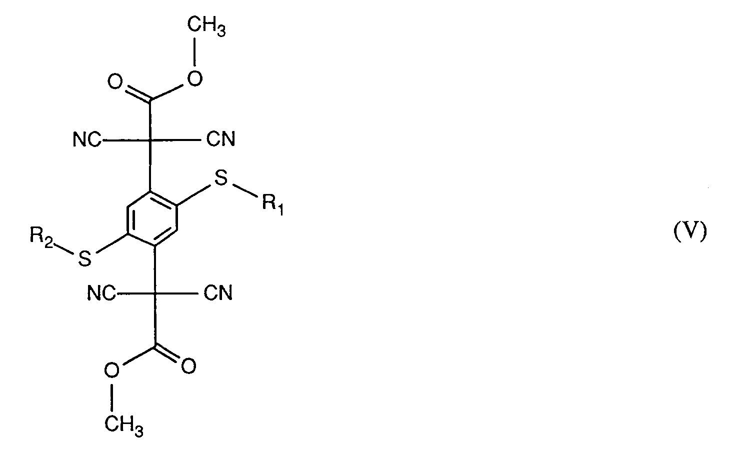 by reaction with CH₃ONa, (CH₃O)₂CO and ClCN, and
iv) dissolving this compound of the formula V in an aqueous base under an inert atmosphere and converting it into the compound of the formula I by the action of Br₂ and concentrated HCl.

7. A compound of the formula III 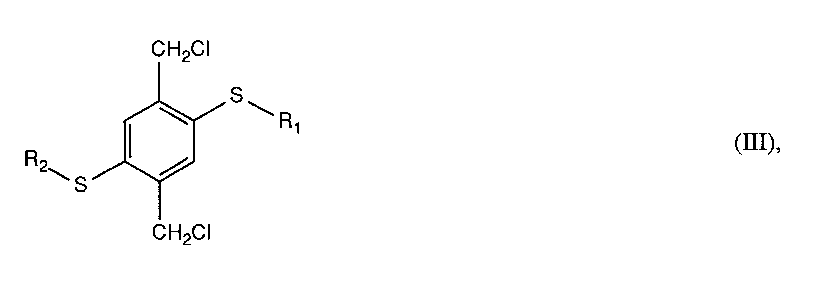 in which
R₁ and R₂ are as defined in claim 1.

8. A compound of the formula III according to claim 7, which is 1,4-bis(chloromethyl)-2,5-bis(thiomethyl)benzene.

9. A carrier material which contains a layer of at least one compound of the formula I according to claim 1 on at least one surface.

10. A material according to claim 9, wherein the carrier is a metal, metal alloy, glass, mineral, ceramic, thermosetting plastic or thermoplastic.

11. A material according to claim 9, wherein the carrier is a glass or homo- or copolymeric plastic.

12. A material according to claim 9, wherein the carrier has a thickness of 0.01 mm to 1 cm.

13. A material according to claim 9, wherein the plastic is a thermoplastic polycarbonate, polyamide, polyester, polyacrylate or polymethacrylate, polyurethane, polyolefin, polyvinyl chloride, polyvinylidene fluoride or polyimide or a thermosetting polyester or epoxy resin.

14. A material according to claim 9, wherein at least one intermediate layer of an inert insulating material is located between the carrier and the layer of a compound of the formula I.

15. A material according to claim 14, wherein the intermediate layer is a plastic.

16. A material according to claim 14, wherein the layer thickness of the intermediate layer(s) is 40 to 3000 Å.

17. A material according to claim 9, wherein 1 to 10 layers of a compound of the formula I are applied to the carrier or the intermediate layer.

18. A material according to claim 17, wherein the layer thickness of the layer(s) of a compound of the formula I is 100 to 3000 Å.

19. A material according to claim 9, wherein a protective layer is located on the layer(s) of a compound of the formula I.

20. The use of a compound of the formula I according to claim 1 for antistatic finishing of objects.

21. The use of the material according to claim 9 as an antistatic packaging material.

## Revendications

1. Composés de formule (I) : 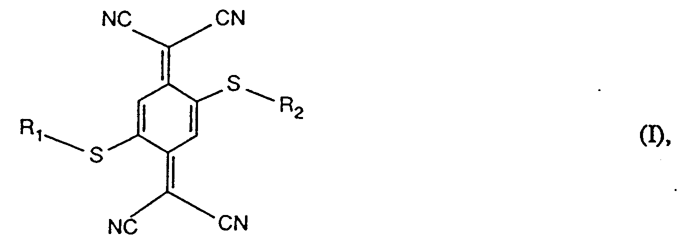 dans laquelle :
R₁ et R₂, indépendamment l'un de l'autre, sont des radicaux alkyle en C₁-C₆, cycloalkyle en C₃-C₈ non substitués ou substitués par des substituants alkyle en C₁-C₄, ou encore phényle ou benzyle, non substitué ou substitué par des substituants F, Cl, Br, alkyle en C₁-C₆, alcoxy en C₁-C₆, alcoxy en C₁-C₆ ou di(alkyle en C₁-C₆)-amino.

2. Composés de formule (I) selon la revendication 1, dans lesquels R₁ et R₂ sont identiques.

3. Composés de formule (I) selon la revendication 1, dans lesquels R₁ et R₂, indépendamment l'un de l'autre, sont des radicaux alkyle en C₁-C₄.

4. Composés de formule (I) selon la revendication 3, dans lesquels R₁ et R₂ sont les radicaux méthyle, éthyle, n-propyle, cyclopentyle, cyclohexyle, phényle ou benzyle.

5. Composé de formule (I) selon la revendication 1, caractérisé en ce qu'il s'agit du 2,5-bis(thiométhyl)-tétracyanoquinodiméthane.

6. Procédé pour préparer des composés de formule (I), caractérisé en ce que :
(i) On fait réagir un composé de formule (II) : 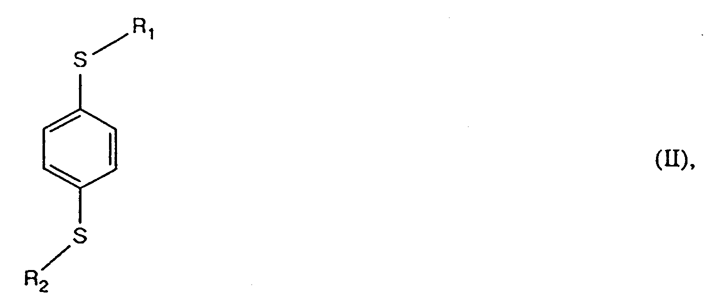 dans laquelle R₁ et R₂ ont les significations données ci-dessus, en présence de HCl concentré et de CH₃COOH, avec du paraformaldéhyde pour obtenir un composé de formule (III) : 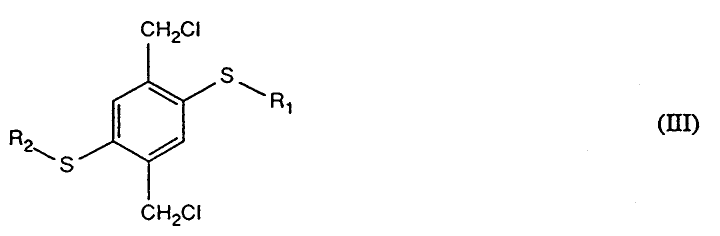
(ii) On convertit ce composé de formule (III), par réaction avec du NaCN, en un composé de formule (IV) : 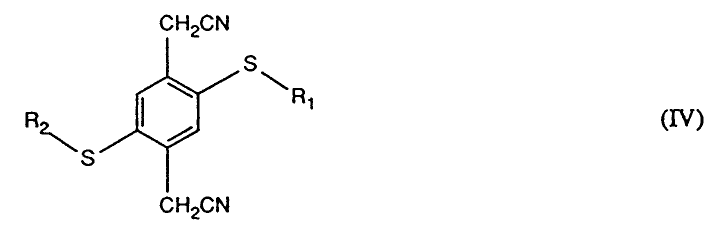
(iii) Puis, par réaction avec du CH₃ONa, du (CH₃O)₂CO et du ClCN, on convertit ce composé de formule (IV) en un composé de formule (V) : 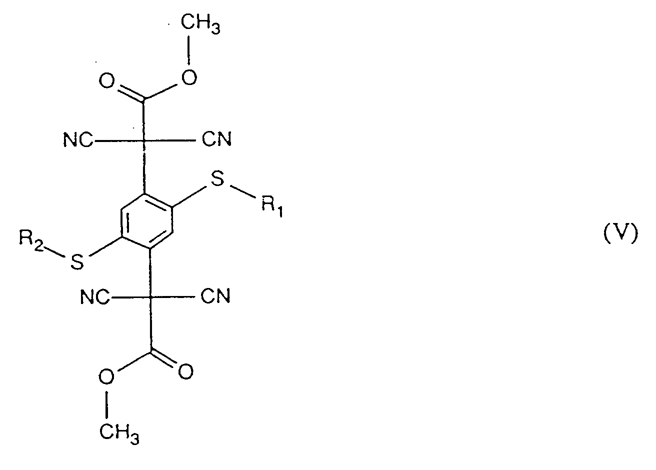 et
(iv) On dissout ce composé de formule (V) dans une base aqueuse sous atmosphère inerte, et, sous l'action de Br₂ et de HCl concentré, on le convertit en le composé de formule (I).

7. Composés de formule (III) : 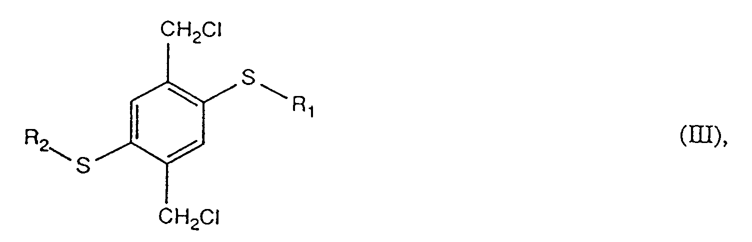 dans laquelle R₁ et R₂ ont les significations données dans la revendication 1.

8. Composé de formule (III) selon la revendication 7, caractérisé en ce qu'il s'agit du 1,4-bis(chlorométhyl)-2,5-bis(thiométhyl)-benzène.

9. Matériau support, qui sur au moins une surface contient une couche d'au moins un composé de formule (I) selon la revendication 1.

10. Matériau selon la revendication 9, caractérisé en ce que, pour ce qui est du support, il s'agit de métaux, d'alliages métalliques, de verres, de produits minéraux, de céramiques et de matériaux plastiques, thermoplastiques ou thermodurcissables.

11. Matériau selon la revendication 9, caractérisé en ce que, pour ce qui est du support, il s'agit de verres et de plastiques homo- ou copolymères.

12. Matériau selon la revendication 9, caractérisé en ce que le support a une épaisseur de 0,01 mm à 1 cm.

13. Matériau selon la revendication 9, caractérisé en ce que, pour ce qui est du plastique, il s'agit des matériaux thermoplastiques polycarbonates, polyamides, polyesters, polyacrylates et polyméthacrylates, polyuréthannes, polyoléfines, poly(chlorure de vinyle), poly(fluorure de vinylidène), polyimides, polyesters thermodurcissables et résines époxydes.

14. Matériau selon la revendication 9, caractérisé en ce qu'au moins une couche intermédiaire, en un matériau isolant inerte, se trouve entre le support et la couche constituée des composés de formule (I).

15. Matériau selon la revendication 14, caractérisé en ce que, pour ce qui est de la couche intermédiaire, il s'agit de matières plastiques.

16. Matériau selon la revendication 14, caractérisé en ce que l'épaisseur de la ou des couches intermédiaires est de 40 à 3000 Å.

17. Matériau selon la revendication 9, caractérisé en ce qu'on applique sur le support ou sur la couche intermédiaire 1 à 10 couches des composés de formule (I).

18. Matériau selon la revendication 17, caractérisé en ce que l'épaisseur de la ou des couches en les composés de formule (I) est de 100 à 3000 Å.

19. Matériau selon la revendication 9, caractérisé en ce qu'une couche de protection se trouve sur la ou les couches constituées de composés de formule (I).

20. Utilisation des composés de formule (I) selon la revendication 1 pour l'apprêt antistatique d'objets.

21. Utilisation du matériau selon la revendication 9 comme matériau d'emballage antistatique.
